Europäisches Patentamt

European Patent Office

Office européen des brevets

(19)

(11) Veröffentlichungsnummer: **0 337 317**
**A1**

## (12) EUROPÄISCHE PATENTANMELDUNG

(21) Anmeldenummer: **89106166.5**

(22) Anmeldetag: **07.04.89**

(51) Int. Cl.⁴: **A61B 5/00**

(30) Priorität: **07.04.88 DE 3811689**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **A. Nattermann & Cie. GmbH**
**Nattermannallee 1**
**D-5000 Köln 30(DE)**

(72) Erfinder: **Blazek, Vladimir, Dr.-Ing.**
**Königshügel 31**
**D-5100 Aachen(DE)**
Erfinder: **Schmitt, Hans J., Prof. Dr.rer.nat.**
**Hangstrasse 34**
**D-5100 Aachen(DE)**
Erfinder: **Mühl, Thomas, Dr.Ing.**
**Behringstrasse 1**
**D-7412 Eningen(DE)**

(74) Vertreter: **Münich, Wilhelm, Dr. et al**
**Kanzlei Münich, Steinmann, Schiller**
**Willibaldstrasse 36/38**
**D-8000 München 21(DE)**

(54) **Optischer Sensor für Langzeitanwendungen bei der nichtinvasiven Messung von Blut-und Durchflussparametern.**

(57) Beschrieben wird ein optischer Sensor zur nicht-invasiven Erfassung mikrozirkulatorischer Blutvolumen-, Blutabfluß-und -durchflußparameter, mit wenigstens einer Lichtemissions- (6) und einer Lichterfassungseinrichtung (7), die in einem Sensorgehäuse (1) angeordnet sind, das auf die Hautoberfläche aufsetzbar ist.

Der erfindungsgemäße Sensor zeichnet sich dadurch aus, daß das Sensorgehäuse (1) Luft-Zirkulationsöffnungen (9,9') aufweist, die einen Luftaustausch zwischen der Hautoberfläche unter den Sensorelement und der umgebenden Luft ermöglichen.

Fig. 1

## Optischer Sensor für Langzeitanwendungen bei der nichtinvasiven Messung von Blut- und Durchfluß-parametern

Die Erfindung bezieht sich auf einen optischen Sensor zur nichtinvasiven (unblutigen) Erfassung diverser Paramenter der Haut, vorzugsweise der hämodynamischen Parameter, wie z.B. Blutgehalt, Blutmenge, Blutgeschwindigkeit.

Seit langem ist z.B. bekannt, daß die Blutfülle in den Gefäßen der menschlichen Haut u.a. rhythmischen Schwankungen unterliegt, in denen sich cardiale, ventilatorische sowie zentralnervös geregelte Rhythmen wiederfinden. Außerdem ist bekannt, daß man aus Änderungen der optischen Eigenschaften der Haut je nach Meßbedingungen Schlüsse z.B. über den peripheren Venendruck ziehen kann.

Dank der modernen optoelektronischen und optischen Komponenten (kalte, quasimonochromatische Lichtquellen, empfindliche Photodetektoren, Lichtwellenleiter erscheint es besonders vorteilhaft, diese Parameter nichtinvasiv und damit für den Menschen völlig schmerz- und risikofrei durch optische Abtastung der Haut zu gewinnen.

Dazu werden neben dem entsprechenden elektronischen Steuer- und Auswertesystem optische Sensoren verwendet, welche an der Haut befestigt werden und einen bzw. mehrere Emissions- und/oder Detektionsvorrichtungen aufweisen. Mit den Emissionsvorrichtungen (LED, LD, LWL) wird die Haut im Meßareal unter dem Sensor mit vorzugsweise moduliertem Licht bestrahlt.

Die in die Haut eingekoppelten Strahlungsanteile werden an verschiedenen Phasengrenzen, wie z.B. Gefäßen reflektiert bzw. gestreut und teilweise wieder aus der Haut zurückgeworfen, so daß sie von der Detektionsvorrichtung (PIN, APD, LWL) erfaßt werden. Aus diesen wiedererfaßten Strahlungsanteilen können schließlich die gesuchten Bioparameter gewonnen werden.

Solche optischen Sensoren sind beispielsweise aus der DE-PS 31 00 610 oder der DE-A1 36 09 075 bekannt.

Diese Sensoren werden zweckmäßigerweise mit beiderseits klebenden Folienringen, Manschetten, Bändern oder Klebestreifen an der Haut fixiert.

Alle Sensoren gemäß dem Stand der Technik haben aber den Nachteil, daß durch die Hautabdeckung mit dem Sensor das thermoregulatorische Gleichgewicht des zugedeckten Hautareals gestört wird, d.h. daß ein sog. Okklusionseffekt stattfindet. Die Haut kann in diesem Bereich nicht "atmen", was in der Regel bereits nach wenigen Minuten zu einer Steigerung der Temperatur, der Hautfeuchtigkeit und schließlich auch der Hautdicke unter dem Sensor führt.

Der für die optische Abtastung der Biosignale erforderliche Zusammenhang zwischen der Makro- und Mikrozirkulation besteht also unter den gestörten thermoregulatorischen Bedingungen nicht mehr, bzw. nicht mehr exakt.

Der Temperaturanstieg im Meßareal resultiert in der Regel aber nicht nur aus der Okklusion der Haut durch den Sensor, auch die Energieverluste der optoelektronischen Wandler oder sonstigen elektronischen Bauteile im Sensorgehäuse bzw. Absorptionsverluste der optischen Strahlung im Gewebe selbst führen zu einem unkontrolliertem Temperaturanstieg.

Diese Effekte können die gewonnen Biosignale derart verfälschen, daß der Einsatz der nach dem Stande der Technik bekannten optischen Sensoren eine Langzeitapplikation praktisch unmöglich macht. Natürlich sind auch die Meßbedingungen denkbar, bei denen durch gegenüber der Hauttemperatur unterkühlten Sensoren ebenfalls Meßfehler entstehen können.

Eine Langzeitapplikation optischer Sensoren erscheint aber gerade bei aktuellen physiologischen Fragestellungen, wie Langzeiterfassung der menschlichen Mikrozirkulationsrhythmik (unter irdischen Bedingungen aber auch bei Mikrogravitation während der Raumflüge) von enormer Bedeutung.

Der Erfindung liegt deshalb die Aufgabe zugrunde, eine einen Sensor der im Oberbegriff des Anspruchs 1 vorausgesetzten Art anzugeben, der das Temperaturgleichgewicht der Haut nicht stört.

Erfindungsgemäß ist erkannt worden, daß es zur Lösung dieser Aufgabe erforderlich ist, Okklusionseffekte bzw. unerwünschte Klimaveränderungen im Meßareal unter dem Sensor zu vermeiden. Darüberhinaus ist erfindungsgemäß aber auch erkannt worden, daß es erforderlich ist, daß die Sensoren auch gezielt die Einstellung eines gewünschten Klimas im Meßareal der Haut dadurch ermöglichen, daß die Möglichkeit für eine Umspülung des Hautareals unter dem Sensor mit Gasen (oder Flüssigkeiten) geschaffen wird.

Ein diese Aufgabe lösender Sensor ist mit seinen Weiterbildungen in den Patentansprüchen gekennzeichnet.

Der erfindungsgemäße Sensor weist ein Sensorgehäuse mit einer oder mehreren Öffnungen auf. Durch diese Öffnungen findet ein natürlicher, passiver Luftaustausch statt, die Haut im Meßareal kann atmen, Okklusionseffekt bzw. Änderung des thermoregulatorischen Gleichgewichts werden vermieden.

Für bestimmte physiologische Fragestellungen kann es aber auch von Interesse sein, das thermoregulatorische Gleichgewicht gezielt zu ändern. Ein Beispiel hierfür ist die Unterkühlung der Haut auf z.B. 18°C. Optische Messungen bei dieser sog. Indifferenz-Temperatur (18°C oder weniger) sind optimal dazu geeignet, bei physiologischer Hautdurchblutung die sog. aktiven Kreislaufrhythmen zu registrieren. Bei zahlreichen pathophysiologischen Zuständen fehlen aber auch bei der Indifferenz-Temperatur diese aktiven Rhythmen, so daß damit ein Nachweis einer krankhaften Hautdurchblutung geführt werden kann (vergl. Schmid-Schönbein, H. et al.: Rhythmische Schwankungen der Blutfülle in der menschlichen Haut: Physiologische Grundlagen und Bedingungen für ihre Registrierung. Biomed. Techn. 30 (erg. B), Sept. 1985, S. 14 - 15).

Die Erfindung wird nachstehend anhand einiger bevorzugten Ausführungsbeispiele unter Bezugnahme auf die Zeichnungen näher beschrieben.

Fig. 1 zeigt einen optischen Sensor mit Belüfungskanälen und optoelektronischen Wandlern

Fig. 2 zeigt einen Sensor mit in die Stirnfläche eingesenkten Belüftungskanälen; Emission- und Detektionsvorrichtungen sind nicht eingezeichnet.

Fig. 3 zeigt ein anderes Ausführungsbeispiel des optischen Sensors ( sog. faseroptischer Sensor) mit Belüftungskanälen und Lichtwellenleiteranschlüssen.

Die Bezugszeichen in diesen Figuren bedeuten durchgehend: —

1 ... Sensor Gehäuse
2 ... Kontaktfläche zur Haut
3 ... Hohlraum an der Stirnseite des Sensors
4 ... Rückwand des Sensors
5 ... Hohlraum im Sensor
6 ... Emissions-Vorrichtung (LED, LD, LWL)
6′... Öffnung für Emissions-Vorrichtung
7 ... Detektions-Vorrichtung (PIN, APD, LWL)
7′... Öffnung für Detektions-Vorrichtung
8 ... Verbindungskabel zum Meß- und Auswertesystem (elektrisch, optisch)
9 ... Lüftungsöffnungen an der Stirnseite des Sensors
9′... Lüftungsöffnungen an der Rückwand des Sensors
10... Schlauch für Be- und Entlüfung
11 ... Pumpe
12 ... Steuerelektronik für die Pumpe

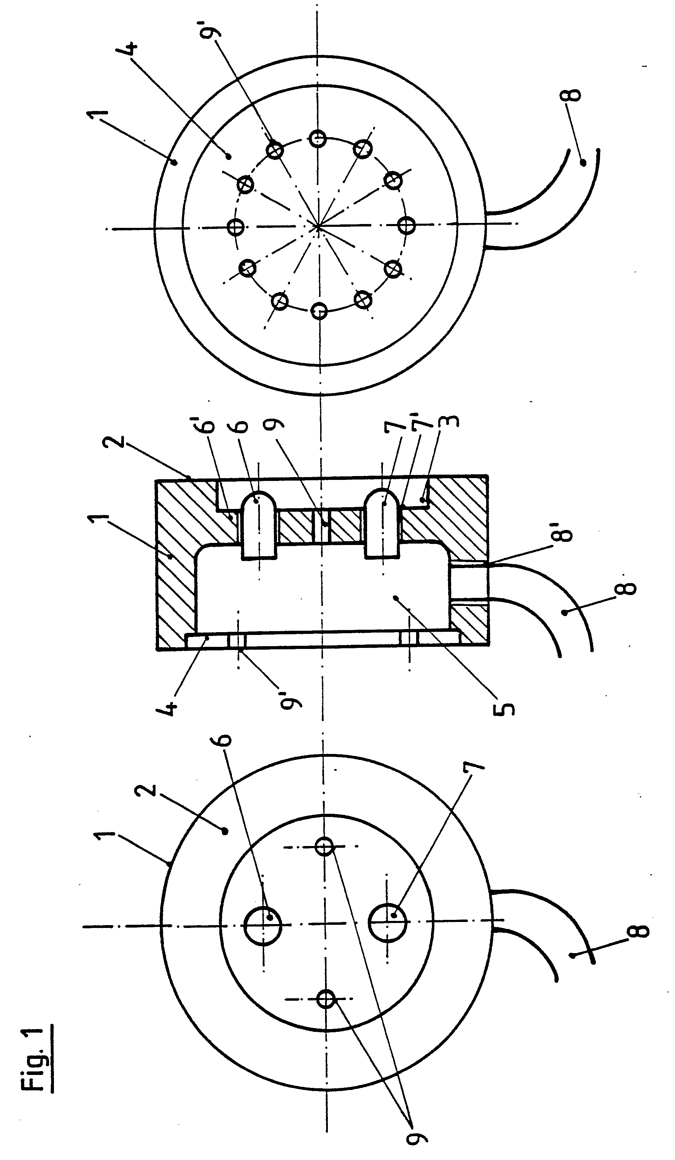

Figur 1 zeigt ein rundes Sensorgehäuse 1. Die Befestigung an die Haut findet vorzugsweise mit einem beiderseits klebendem Ring im Bereich der Kontaktfläche 2 statt. Damit entsteht ein Hohlraum 3 zwischen der Haut und der Stirnseite des Sensors. Die Emissions- und Detektionvorrichtung 6 und 7 sind vorzugsweise senkrecht zur Hautoberfläche ausgerichtet, sie sind in den entsprechenden Öffnungen 6′ und 7′ befestigt. Bei diesem Ausführungsbeispiel ist im Sensorgehäuse ein weiterer Hohlraum 5 vorgesehen, der zum Unterbringen von beispielsweise Vorverstärker-Schaltkreisen dienen kann. Durch die Öffnungen 9 und 9′ erfolgt eine "Atmung" der Haut durch das Sensorgehäuse 1 und dessen Rückwand 4. Damit wird das thermoregulatorische Gleichgewicht der Haut im Meßareal gewährleistet. Außerdem können die evtl. vorhandenen Wärmeemissionen in optoelektronkschen und/oder elektronischen Bauteilen im Sensorgehäuse über die Öffnungen 9′ in der Rückwand 4 besser nach außen abgeführt werden, was ebenfalls für die o.e. Zielsetzung von Bedeutung ist.

Außerdem zeigt die Fig. 1 das Verbindungskabel 8 (mit der Öffnung 8′) zum Abschluß des Sensors an die Meß- und Auswerteelektronik.

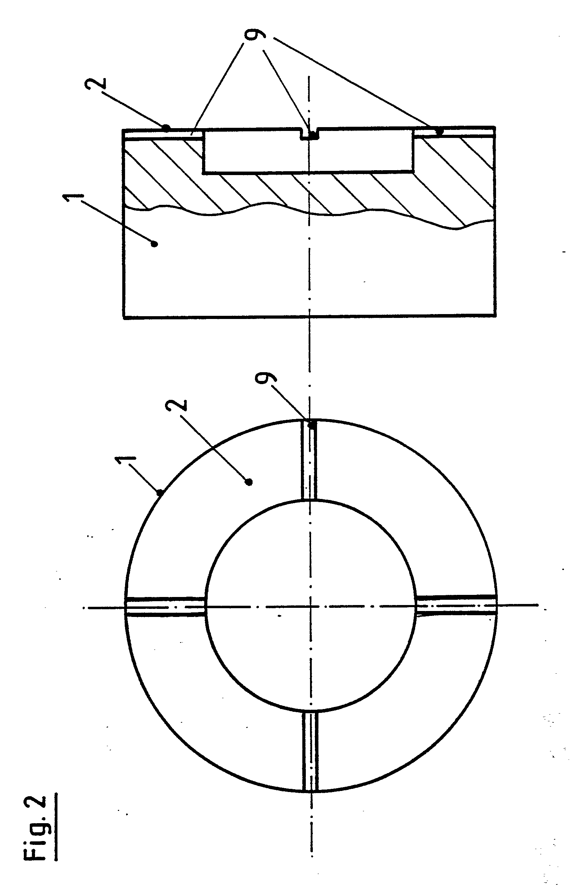

Die Fig. 2 zeigt eine andere Ausführungsform des erfindungsgemäßen Sensors. Sie wird bevorzugt in dem Fall, daß eine axiale Luftführung durch den Sensor nicht möglich ist (z.B. wenn der Hohlraum 5 mit Elektronik gefüllt ist). Hierzu werden in die Kontaktfläche 2 radial Gräten als Belüftungskanäle eingearbeitet, die dann die Funktion der Luftzirkulation übernehmen.

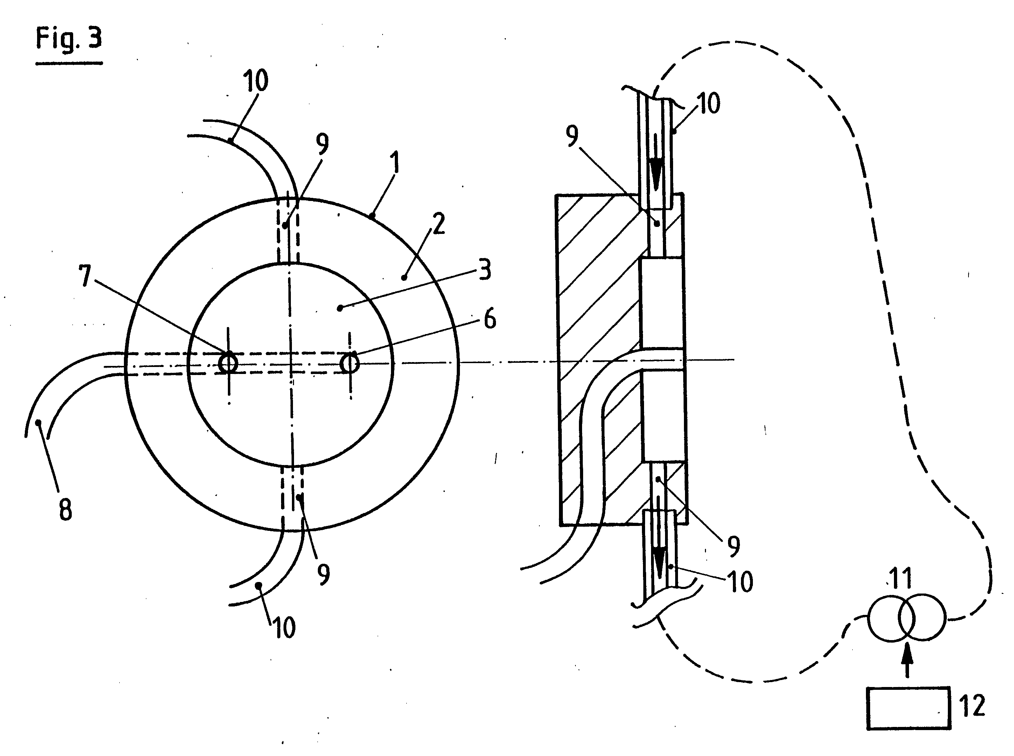

Fig. 3 zeigt einen sog. faseroptischen Sensor. Die Emissions- und Detektionsvorrichtungen sind als Stirnflächen von Lichtwellenleitern ausgebildet. Die Belüftungskanäle 9 sind derart ausgebildet, daß auch Schläuch 10 zur Verbin dung des Sensors mit einer externen Pumpe 11 möglich wird. Dadurch kann die Luftzirkulation auch aktiv durchgeführt werden.

In diesem Fall kann die Haut im Meßareal beliebig temperiert werden, d.h. das thermoregulatorische Gleichgewicht der Haut kann gezielt und kontrolliert verändert werden. Als Klimamedium dient vorzugsweise Luft. Aber auch andere Gase oder gar Flüssigkeiten sind denkbar. Die Steuerung der Pumpe übernimmt dann eine Steuerungselektronik 12, die fur eine gezielte Klimaeinstellung in diesem geschlossenen, aktiven Klimakreis sorgt.

Die Steuerung kann eine handelsübliche Mikroprozessorschaltung sein. Ferner ist es auch möglich, daß die Auswerteelektronik die Steuerung der Pumpe "mit übernimmt".

## Ansprüche

1. Optischer Sensor zur nichtinvasiven Erfassung mikrozirkulatorischer Blutvolumen-, Blutabfluß- und -durchflußparameter, mit wenigstens einer Lichtemissions- und einer Lichterfassungseinrichtung, die in einem Sensorgehäuse angeordnet sind, das auf die Hautoberfläche aufsetzbar ist,
dadurch **gekennzeichnet**, daß das Sensorgehäuse Luft-Zirkulationsöffnungen aufweist, die einen Luftaustausch zwischen der Hautoberfläche unter den Sensorelement und der umgebenden Luft ermöglichen.

2. Sensor nach Anspruch 1, dadurch **gekennzeichnet**, daß die Lüftungsöffnungen axial durch das Sensorgehäuse derart geführt sind, daß durch die Lüftströmung zusätzlich zur Atmung der Haut auch die Abkühlung von elektronischen und/oder neuartigen Bauelementen im Sensorgehäuse ermöglicht wird.

3. Sensor nach Anspruch 1, dadurch **gekennzeichnet**, daß die Lüftungsöffnungen direkt in den Stirnflächen des Sensors als Gräten eingearbeitet sind.

4. Sensor nach Anspruch 2, dadurch **gekennzeichnet**, daß an die Lüftungsöffnungen im Sensorgehäuse Schläuche und/oder sonstige Klimakanäle anschließbar sind, die zu einer externen Umwälzeinrichtung und insbesondere einer Pumpe führen, die eine aktive Unterstützung der Luftzirkulation im geschlossenen Klimakreis gewährleistet.

5. Sensor nach einem der Ansprüche 1 bis 4, dadurch **gekennzeichnet**, daß eine elektronische Steuerschaltung die externe Pumpe derart ansteuert, daß gewünschte Klimaparameter an der Hautoberfläche im Meßareal (z.B. Temperatur, Feuchte, Durchflußmenge) gezielt geregelt werden können.

6. Sensor nach einem der Ansprüche 1 bis 5, dadurch **gekennzeichnet**, daß Gase und/oder Flüssigkeiten das Klimamedium bilden.

## Fig. 1

EP 0 337 317 A1

# Fig. 2

EP 0 337 317 A1

# Fig. 3

EP 0 337 317 A1

## Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 446 115 (DRÄGERWERKE AG) * Seite 3, Zeilen 28-55; Seite 4, Zeilen 12-33; Figur * --- | 1,2 | A 61 B 5/00 |
| A | DE-U-8 134 303 (HEWLETT-PACKARD) * Seite 4, Zeile 1 - Seite 5, Zeile 25; Figur 1 * --- | 4-6 | |
| D,A | DE-A-3 100 610 (V. BLAZELE et al.) * Spalte 5, Zeile 25 - Spalte 6, Zeile 26; Figur 1 * --- | 1 | |
| A | US-A-4 537 197 (J.F. HULKA) * Spalte 2, Zeilen 1-51; Figur 2 * ----- | 3 | |

| RECHERCHIERTE SACHGEBIETE (Int. Cl.4) |
|---|
| A 61 B |

Der vorliegende Recherchenbericht wurde für alle Patentansprüche erstellt

| Recherchenort | Abschlußdatum der Recherche | Prüfer |
|---|---|---|
| DEN HAAG | 13-07-1989 | HUNT,B.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)